# EUROPÄISCHE PATENTSCHRIFT

(11) **EP 0 758 884 B1**
(45) Veröffentlichungstag und Bekanntmachung des Hinweises auf die Patenterteilung: **20.12.2000**
(21) Anmeldenummer: 95920805.9
(22) Anmeldetag: 08.05.1995
(51) Int. Cl.: A61K 9/20

(54) **VERFAHREN ZUR HERSTELLUNG VON VORRICHTUNGEN ZUR KONTROLLIERTEN FREISETZUNG VON WIRKSTOFFEN**
ACTIVE SUBSTANCE CONTROLLED RELEASE DEVICES AND PROCESS FOR PRODUCING THE SAME
DISPOSITIFS DE LIBERATION CONTROLEE DE PRINCIPES ACTIFS ET LEUR PROCEDE DE PRODUCTION

(30) Priorität: 13.05.1994 DE 4416926
(43) Veröffentlichungstag der Anmeldung: 26.02.1997
(60) Teilanmeldung: 99114947.7
(73) Patentinhaber: LTS LOHMANN Therapie-Systeme GmbH, 56567 Neuwied (DE)
(72) Erfinder: CREMER, Karsten, 53119 Bonn (DE)
(74) Vertreter: Flaccus, Rolf-Dieter, Dr.
(86) Internationale Anmeldenummer: EP9501725
(87) Internationale Veröffentlichungsnummer: WO9531185

(56) Entgegenhaltungen:
- WO-A-94/07470
- DE-A- 4 341 442

## Beschreibung

Die Erfindung bezieht sich auf ein Verfahren zur Herstellung von Vorrichtungen zur kontrollierten Freisetzung von Wirkstoffen, bestehend aus einer wirkstoffhaltigen Matrix und einer die Matrix mindestens teilweise bedeckenden erodierbaren Feststoffmasse, wobei die Dicke der erodierbaren Masse über deren Erstreckung durch Dickegradienten bestimmt ist, wobei die wirkstoffhaltige Matrix selbst mit Dickegradienten erzeugt wird, welche die Dickegradienten der erodierbaren Masse bestimmen.

Vorrichtungen dieser Art sind in der Patentanmeldung DE-A-4341442 beschrieben. Sie dienen dazu, Wirkstoffe in flüssigen Medien wie z.B. Körperflüssigkeiten kontrolliert, d.h. mit vorgegebenem Geschwindigkeitsprofil freizusetzen. Ein wichtiges Einsatzgebiet dieser Vorrichtungen ist die perorale Verabreichung von Arzneistoffen. Hier ist häufig das Ziel, Wirkstoffe mit kurzer biologischer Halbwertzeit so zu verabreichen, daß bei geringer Einnahmefrequenz gleichmäßige Plasmakonzentrationen aufrechterhalten werden.

Das Geschwindigkeitsprofil der Wirkstofffreisetzung wird bei diesen Vorrichtungen insbesondere von den Dickegradienten der erodierbaren Feststoffmasse mitbestimmt, welche die wirkstoffhaltige Matrix zunächst zumindest teilweise bedecken. Im Laufe der Freisetzung kommt es zu einer voranschreitenden Erosion dieser Feststoffmasse, wodurch sich in Abhängigkeit von ihren Dickegradienten die Kontaktfläche zwischen der wirkstoffhaltigen Matrix und dem flüssigen Medium, in welchem sich die Vorrichtung befindet, vergrößert. Mittels dieses Effektes läßt sich bei entsprechender Wahl der Dikegradienten beispielsweise eine relativ konstante Freisetzungsgeschwindigkeit aufrechterhalten, wie es bei Retardarzneimitteln häufig erwünscht ist.

Aufgrund des neuartigen Konstruktionsprinzips finden sich im Stand der Technik bisher kaum Angaben zu Herstellungsmöglichkeiten für Vorrichtungen dieser Art. Lediglich in der WO 94/07470 wird die Erzeugung von erodierbaren Überzügen unterschiedlicher Dicke durch herkömmliche Beschichtungstechniken in der Rolltrommel oder durch Sprüh- oder Wirbelschichttechniken erwähnt. Auf diese Weise scheint jedoch eine einfache und flexible Erzeugung einer wirkstoffhaltigen Matrix mit einer erodierbaren Feststoffmasse mit vorbestimmbaren Dickegradienten kaum möglich. Der Erfindung liegt daher die Aufgabe zugrunde, ein Verfahren zur Herstellung der Vorrichtungen zu schaffen, welches eine Lösung für dieses Problem enthält und effizient ist.

Diese Aufgabe wird erfindungsgemäß dadurch gelöst, daß in einem ersten Schritt die wirkstoffhaltige Matrix in Form von Preßlingen mit Dickegradienten geformt und in einem zweiten Schritt auf eine Manteltablettenpresse übertragen und dort mit einer erodierbaren Masse zusammengepreßt wird.

Dieses Verfahren läßt sich mit den in der pharmazeutischen Verfahrenstechnik häufig bevorzugten Tablettiertechniken verwirklichen. Dazu wird zunächst ein wirkstoffhaltiger Preßling gefertigt, welcher die wirkstoffhaltige Matrix darstellt. Vorzugsweise ist die Form dieses Preßlings bereits auf die erwünschten Dickegradienten der erodierbaren Feststofmmasse abgestimmt, was dadurch möglich ist, daß der Preßling selbst Dickegradienten enthält, welche tendenziell komplementär zu den Dickegradienten der erodierbaren Feststoffmasse sind. Diese Preßlinge werden auf Manteltablettenpressen übertragen, auf denen sie mit der erodierbaren Masse zusammengepresst werden. Die Verwendung von Mantelkernpressen ist hierfür besonders vorteilhaft, weil sie bei entsprechender Konstruktion dazu in der Lage sind, die vorgefertigten Preßlinge automatisch und kontinuierlich in die Matrizenbohrungen zu transferieren, in denen der zweite Preßvorgang stattfindet.

Vorrichtungen der o.g. Art nach dem hier beschriebenen Verfahren durch Tablettieren herzustellen, ist insbesondere für die Fertigung großer Stückzahlen sehr wirtschaftlich. Dabei könnte ein Matrix-Preßling auch durch Anteigen von pulverförmigen Materialien bis zur pastösen Konsistenz und Extrusion gewonnen und wie beschrieben weiterverarbeitet werden.

Beim erfindungsgemäßen Verfahren zur Herstellung von Vorrichtungen der o.g. Art kann auch beispielsweise zunächst eine wirkstoffhaltige "Roh-Matrix" durch einen oder mehrere hintereinandergeschaltete Beschichtungsprozesse hergestellt werden, in die dann unter Preßdruck durch Stempel Vertiefungen zur Aufnahme der erodierbare Masse geprägt werden.

Es kann sich als notwendig erweisen, vor dem Zusammenbringen der wirkstoffhaltigen Matrix mit der erodierbaren Masse einen oder mehrere adhäsionsfördernde Zusätze auf die Kontaktfläche zwischen den einzelnen Komponenten der Vorrichzung aufzutragen. Die Auftragung der klebenden Hilfsstoffe geschieht vorzugsweise dadurch, daß eine Lösung dieser Stoffe vorliegt, die in einem Beschichtungs-, Sprüh- oder Tauchverfahren appliziert wird.

Unabhängig von der Ausgestaltung des Herstellungsverfahrens Unabhängig von der Ausgestaltung des Herstellungsverfahrens gelten alle beschriebenen Optionen auch für den Fall, daß Vorrichtungen zur kontrollierbaren Freisetzung von Wirkstoffen mit mehr als einer wirkstoffhaltigen Matrix und einer die Matrices zumindest teilweise bedeckenden, erodierbaren Feststoffmasse hergestellt werden sollen.

## Patentansprüche

1. Verfahren zur Herstellung von Vorrichtungen zur kontrollierten Freisetzung von Wirkstoffen, bestehend aus einer wirkstoffhaltigen Matrix und einer die Matrix mindestens teilweise bedeckenden erodierbaren Feststoffmasse, wobei die Dicke der erodierbaren Masse über deren Erstreckung durch Dickegradienten bestimmt ist, wobei die wirkstoffhaltige Matrix selbst mit Dickegradienten erzeugt wird, welche die Dickegradienten der erodierbaren Masse bestimmen, dadurch gekennzeichnet, daß in einem ersten Schritt die wirkstoffhaltige Matrix in Form von Preßlingen mit Dickegradienten geformt und in einem zweiten Schritt auf eine Manteltablettenpresse übertragen und dort mit einer erodierbaren Masse zusammengepreßt wird.

2. Verfahren nach Anspruch 1, dadurch gekennzeichnet, daß die wirkstoffhaltige Matrix durch Stempeln oder Walzen Vertiefungen erhält, welche ihre Dickegradienten bestimmen.

3. Verfahren nach Anspruch 1 oder 2, dadurch gekennzeichnet, daß die Vorrichtung zur kontrollierten Freisetzung von Wirkstoffen mit mehr als einer wirkstoffhaltigen Matrix hergestellt wird.

4. Verfahren nach einem oder mehreren der Ansprüche 1 bis 3, dadurch gekennzeichnet, daß vor dem Zusammenbringen von wirkstoffhaltiger Matrix und erodierbarer Masse ein adhäsionsfördernder Hilfsstoff auf die Kontaktfläche zwischen den beiden Komponenten aufgebracht wird.

## Claims

1. A process for the production of devices for the controlled release of active substances, consisting of an active substance-containing matrix and an erodible mass of solids at least partially covering the matrix, with the thickness of the erodible mass across its extension being determined by thickness gradients, and the active substance-containing matrix itself being produced with thickness gradients which determine the thickness gradients of the erodible mass, characterized in that in a first step the active substance-containing matrix is formed in the form of pressed articles having thickness gradients, and in a second step it is transferred onto a dry-coated tablet-compressing machine where it is compressed with the erodible mass.

2. The process according to Claim 1, characterized in that the active substance-containing matrix is provided, by means of stamping or rolling, with depressions, which determine the thickness gradients of the erodible mass.

3. The process according to Claim 1 or 2, characterized in that the device for the controlled release of active substances is produced with more than one active substance-containing matrix.

4. The process according to one or more of claims 1 to 3, characterized in that prior to the combination of active substance-containing matrix and erodible mass, an adhesion promoting auxiliary is applied to the contact surface between the two components.

## Revendications

1. Procédé pour la préparation de dispositifs destinés à la libération contrôlée de substances actives, constitués par une matrice contenant une substance active et par une matière de substance solide apte à être érodée recouvrant la matrice au moins en partie, dans lequel l'épaisseur de la matière apte à être érodée est déterminée, sur son étendue, par des gradients d'épaisseurs, la matrice contenant une substance active étant elle-même obtenue avec des gradients d'épaisseurs qui déterminent les gradients d'épaisseurs de la matière apte à être érodée, caractérisé en ce qu'on façonne dans une première étape la matrice contenant une substance active sous ta forme de comprimés possédant des gradients d'épaisseurs et, dans une seconde étape, on les transfère à une machine de fabrication de comprimés enrobés et on les y comprime avec une matière apte à s'éroder.

2. Procédé selon la revendication 1, caractérisé en ce que l'on obtient la matrice contenant des substances actives par estampage ou par laminage d'enfoncements qui déterminent ses gradients d'épaisseur.

3. Procédé selon la revendication 1 ou 2, caractérisé en ce qu'on prépare le dispositif pour ta libération contrôlée de substances actives avec plus d'une matrice contenant une substance active.

4. Procédé selon une ou plusieurs des revendications 1 à 3, caractérisé en ce que, avant d'amener la matrice contenant une substance active en contact avec la matière apte à s'éroder, on applique un adjuvant favorisant l'adhérence, sur la surface de contact entre les deux composants.
